# FASCICULE DE BREVET EUROPEEN

(11) **EP 1 027 091 B1**
(45) Date de publication et mention de la délivrance du brevet: **11.02.2004**
(21) Numéro de dépôt: 98947598.3
(22) Date de dépôt: 05.10.1998
(51) Int. Cl.: A61M 29/02

(54) **CATHETER UNIVERSEL**
UNIVERSALKATHETER
MULTIPURPOSE CATHETER

(30) Priorité: 31.10.1997 FR 9713988
(43) Date de publication de la demande: 16.08.2000
(73) Titulaire: Soprane S.A., 69006 Lyon (FR); Watrelot, Antoine, 69006 lyon (FR)
(72) Inventeur: WATRELOT, Antoine, F-69008 Lyon (FR); LOUBENS, Thierry, F-69003 Lyon (FR)
(74) Mandataire: Schmitt, John
(86) Numéro de dépôt international: PCT/FR1998/002119
(87) Numéro de publication internationale: WO 1999/022800

(56) Documents cités:
- EP-A- 0 277 364
- EP-A- 0 495 263
- WO-A-92/17236
- WO-A-97/37716
- FR-A- 2 607 707
- US-A- 4 670 009
- US-A- 5 183 464
- US-A- 5 431 676
- US-A- 5 437 637
- US-A- 5 487 730
- US-A- 5 514 092
- US-A- 5 569 183
- US-A- 5 662 607

## Description

La présente invention est relative à un cathéter universel à usage unique permettant plus particulièrement l'introduction dans le corps humain au niveau du site opératoire, soit des mandrins chirurgicaux, soit l'injection de liquide tel que du sérum ou du bleu de méthylène pour réaliser différents tests préopératoires et peropératoires.

On entend par cathéter universel un cathéter/trocart ayant la fonction d'un outil pour la perforation des parois et la fonction d'un cathéter pour l'introduction dans le site opératoire, soit de produits liquides, soit une gamme de mandrins chirurgicaux ou autres instruments, soit les deux en même temps.

On connaît des cathéters de ce genre qui comportent un corps cylindrique solidaire à l'une de ses extrémités d'une tête munie de deux voies d'accès ou entrées, tandis que l'autre extrémité est pourvue d'un ballon susceptible de se déformer élastiquement sous l'effet d'une pression voir par exemple le document WO92/17236

Ce genre de cathéter permet de réaliser certains tests ou opérations, mais ne peut pas simultanément recevoir des mandrins chirurgicaux, et l'injection de liquides ou le passage d'un mini endoscope du fait que les canaux ménagés dans le corps cylindrique sont coaxiaux entre eux et centrés par rapport aux axes principaux dudit corps.

En effet, un premier canal, centré autour des axes principaux du corps, permet la mise en place d'un mini endoscope ou le passage d'un liquide ou la mise en place d'un mandrin chirurgical. L'ensemble de ses éléments étant introduit séparément et de manière indépendante. Le second canal coaxial au premier est prévu uniquement pour déformer élastiquement le ballon.

On connaît également d'après le brevet US 5 437 637 un cathéter comportant un corps cylindrique souple percé dans sa partie interne de plusieurs canaux non coaxiaux pour le passage de raidisseurs, d'instruments et/ou de liquides. Ce cathéter comprend sur sa périphérie externe un ballonnet qui peut se dilater sous l'effet d'une pression.

On remarque que ce genre de cathéter ne permet pas, du fait de sa souplesse, d'être utilisé comme un trocart afin de perforer des parois. De plus, les moyens de connexion permettent l'introduction dans chaque canal non coaxial, soit d'instruments, soit de liquides, mais ne permettent pas au chirurgien une tenue du cathéter pour pouvoir l'utiliser comme un outil.

C'est à ces inconvénients qu'entend plus particulièrement remédier la présente invention, défini par les revendications 1 à 33.

La description qui va suivre en regard des dessins annexés, donnés à titre d'exemples non limitatifs, permettra de mieux comprendre l'invention, les caractéristiques qu'elle présente et les avantages qu'elle est susceptible de procurer:
Figure 1 est une vue générale représentant le cathéter universel suivant la présente invention.
Figure 2 est une coupe illustrant une première variante du cathéter dont les entrées de la tête débouchent dans des canaux du corps cylindrique.
Figure 3 et 4 sont des coupes suivant III-III et IV-IV de figure 2 montrant la position des canaux ménagés dans le corps cylindrique du cathéter suivant la première variante.
Figure 5 est une coupe représentant une seconde variante du cathéter universel suivant la présente invention.
Figures 6 et 7 sont des coupes suivant VI-VI et VII-VII de figure 5 illustrant une autre configuration des canaux ménagés dans le corps cylindrique du cathéter.
Figure 8 est une vue montrant une troisième variante du cathéter universel en ce qui concerne plus particulièrement le profil de la tête de connexion.
Figures 9 et 10 sont des coupes représentant la tête de connexion suivant la figure 8 et plus particulièrement la position des entrées indépendantes qui coopèrent avec les canaux non coaxiaux du cathéter universel.
Figure 11 est une coupe illustrant le profil de l'extrémité du cathéter universel opposé à celle de la tête de connexion, et la position et le profil du ballonnet.
Figure 12 est une coupe semblable à celle de figure 11 montrant un autre profil du ballonnet.
Figures 13a, 13b et 14a, 14b sont des vues montrant des mandrins prévus pour coopérer avec les canaux du cathéter lors de différentes opérations.
Figure 15 est une vue illustrant un exemple d'utilisation du cathéter universel suivant la présente invention.
Figure 16 est une vue semblable à celle de figure 15, mais représentant un autre exemple d'utilisation du cathéter suivant la présente invention.
Figure 17 est une vue montrant l'utilisation du cathéter universel comme un trocart au cours d'une coelioscopie.
Figure 18 est une vue représentant une autre application concernant l'utilisation du cathéter universel suivant l'invention.

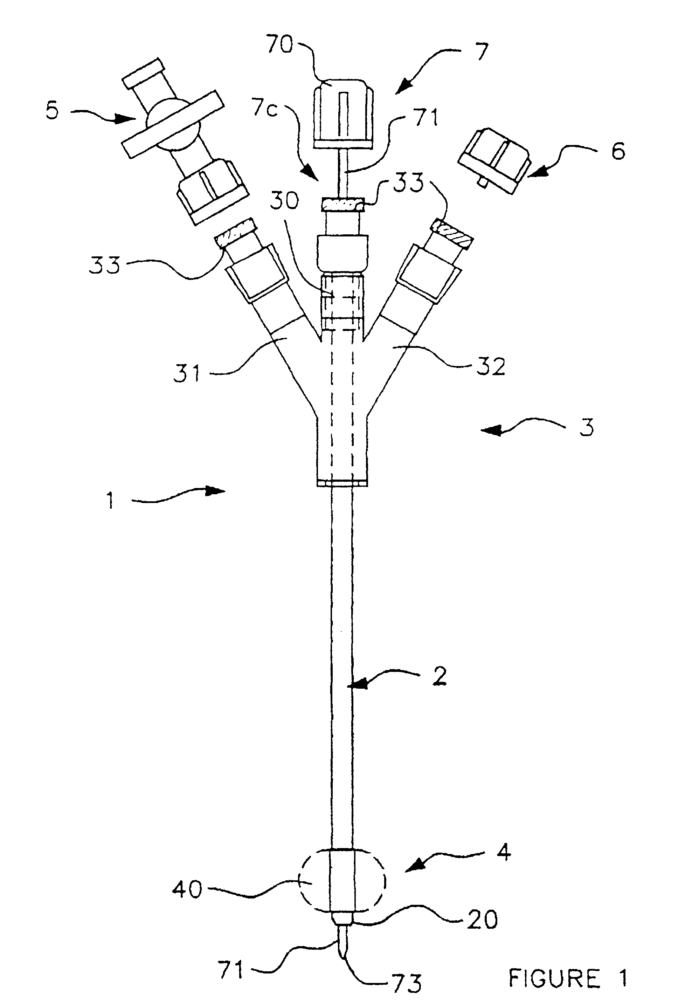

On a montré en figure 1 un cathéter 1 constitué d'un corps cylindrique 2 en matière plastique translucide sensiblement souple ou rigide solidaire à l'une de ses extrémités d'une tête 3 formant des moyens de connexion et comportant trois entrées indépendantes 30, 31 et 32.

Le corps cylindrique 2 présente à l'extrémité opposée à celle portant la tête 3, des moyens d'étanchéité 4 constitués d'un ballon ou ballonnet 40 susceptible de se déformer élastiquement sous l'effet d'une pression.

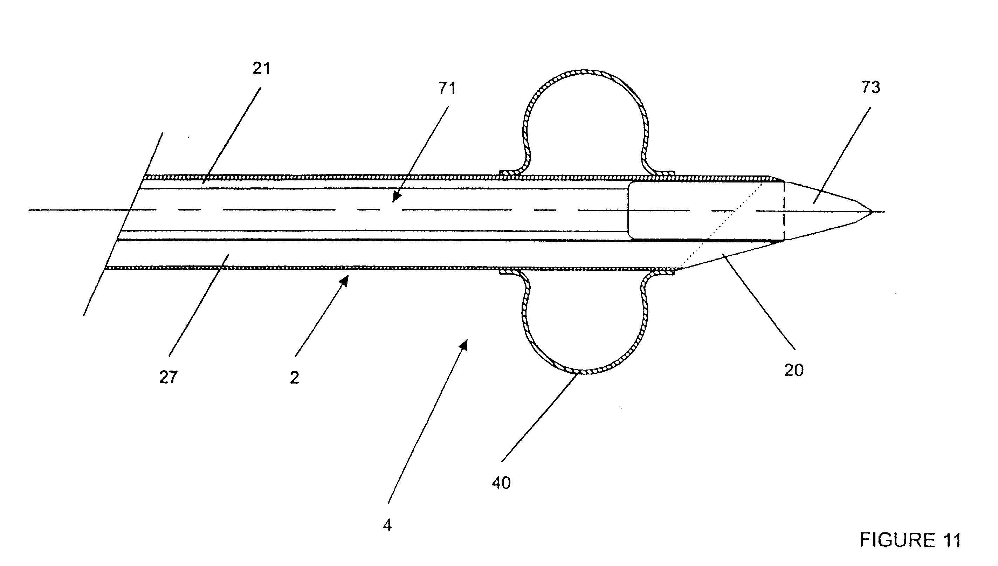

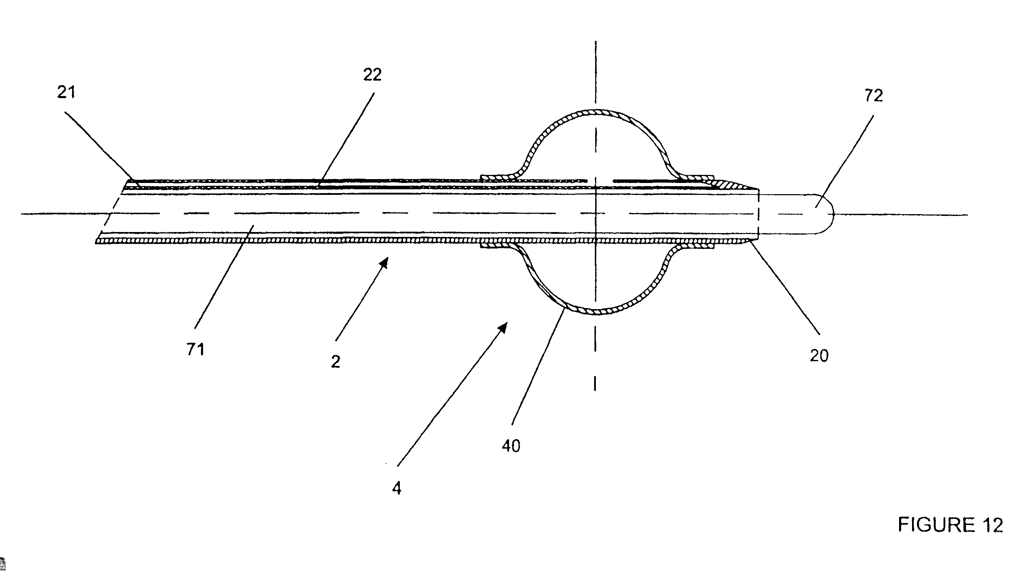

A proximité du ballon 40, l'extrémité libre du corps cylindrique se termine par un profil effilé 20 en forme de pointe (figures 11, 12).

La tête de connexion 3 est réalisée en une matière plastique rigide qui peut être transparente ou non et dont la partie interne est creuse de manière que chaque entrée 30, 31 et 32 débouche dans des canaux ménagés dans le corps cylindrique 2.

La tête de connexion 3 est monobloc et présente un profil de fourche en double Y, c'est à dire que l'entrée 30 est portée par le même axe longitudinal que celui du corps cylindrique 2, tandis que les deux autres entrées 31 et 32 sont décalées latéralement pour être disposées respectivement de chaque côté de l'entrée 30.

Les entrées 30, 31 et 32 sont solidaires chacune d'un élément de liaison 33 permettant la fixation étanche, par exemple, d'une vanne 5 pour l'introduction de liquides, d'un bouchon 6 obstruant une entrée, et la mise en place d'une série de mandrins chirurgicaux 7 dont le profil varient suivant le cas de l'intervention.

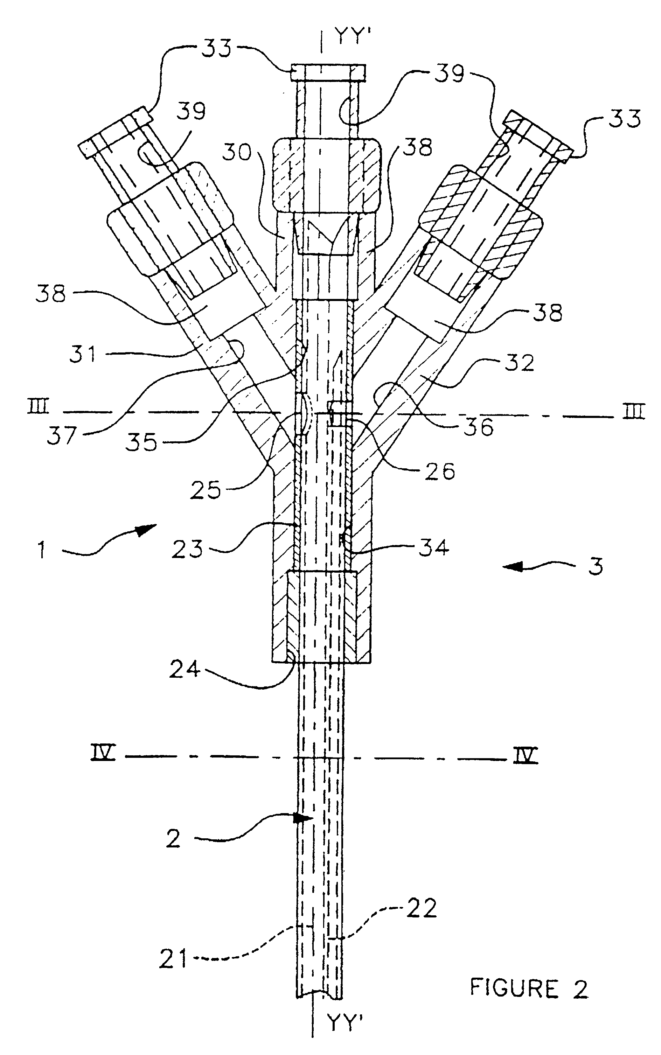

En figures 2 à 4 on a représenté une première variante du cathéter universel 1 dont le corps cylindrique 2 est percé, sur sa longueur, et suivant des axes longitudinaux décalés par rapport à ceux principaux XX' et YY' de deux canaux 21 et 22.

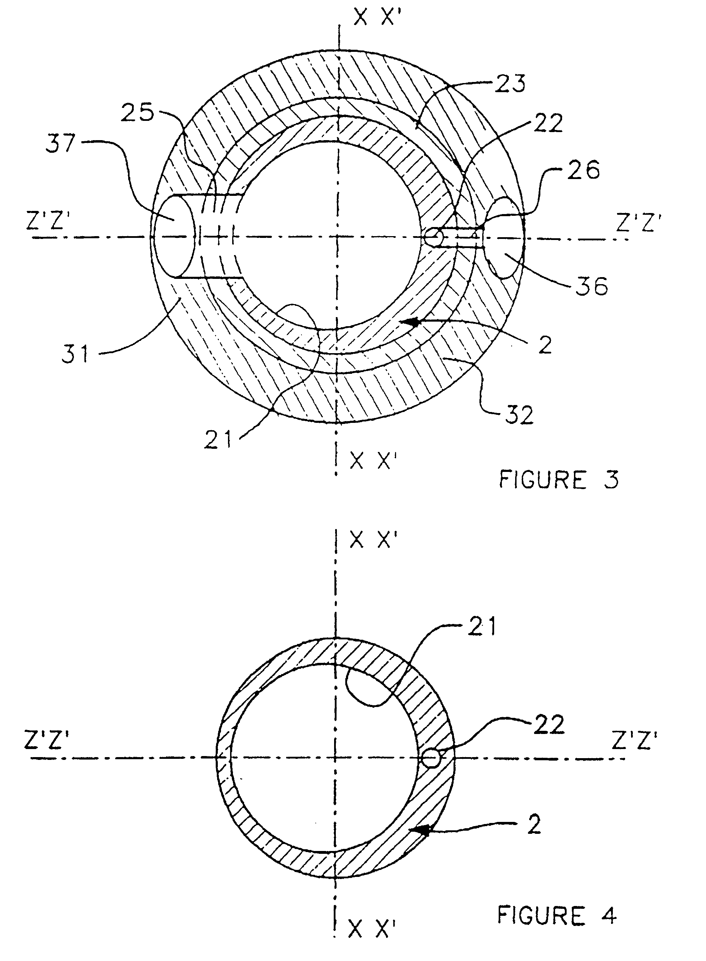

On note que le diamètre du canal principal 21 est supérieur à celui prévu pour le canal 22 et que ces derniers sont portés par au moins un axe principal ZZ' du corps cylindrique 2 (figure 4). Egalement, les canaux 21 et 22 sont décalés l'un par rapport à l'autre. De plus, le canal 21 est excentré par rapport au diamètre extérieur du corps cylindrique 2.

La tête 3 présente un alésage interne 34 qui débouche dans chaque entrée 30, 31 et 32 par l'intermédiaire d'un alésage 35, 36 et 37. Les entrées 30, 31 et 32 comportent chacune une chambre 38 dans laquelle débouche, d'une part chaque alésage 35, 36 et 37, et d'autre part les éléments de liaison 33.

Les éléments de liaison 33 présentent un alésage interne 39 qui communique avec la chambre 38 de chaque entrée 30, 31 et 32 de la tête 3.

La tête 3 est solidaire du corps cylindrique 2 de manière étanche au moyen d'une première bague 24 qui est disposée autour dudit corps et à l'opposé de l'entrée 30 dans un épaulement de l'alésage 34 qui est prévu d'un diamètre supérieur à ce dernier.

Une seconde bague 23 est placée autour du corps cylindrique 2 dans le prolongement de la première de manière à coopérer avec les alésages 34 et 35 de la tête 3. Les bagues 23 et 24 sont collées sur le profil externe du corps cylindrique 2 et sur le pourtour interne des alésages 34 et 35 afin de rendre la tête de connexion 3 solidaire dudit corps.

Les deux bagues 23 et 24 sont prévues de diamètres extérieurs différents, tandis que leur diamètre interne est identique pour coopérer coaxialement autour du corps cylindrique 2.

Le corps cylindrique 2 traverse la chambre 38 de l'entrée 30 pour permettre au canal de grand diamètre 21 de déboucher dans l'alésage 39 de l'élément de liaison 33 correspondant.

A l'opposé, le canal principal 21 débouche à l'extérieur du corps cylindrique 2 suivant le profil effilé 20 en forme de pointe ou conique.

On remarque que l'extrémité 20 du corps cylindrique 2 présente un profil conique qui est axé sur le canal principal 21. Ce dernier est excentré par rapport au diamètre extérieur du corps cylindrique 2, ce qui donne ce profil particulier à l'extrémité 20 (figures 11, 12).

Ainsi, l'extrémité 20 présente un profil conique qui est d'une inclinaison d'environ 30° degrés.

Le profil conique de l'extrémité 20 est prévu pour venir dans le prolongement des instruments ou mandrins 7 dont l'extrémité est également conique.

On constate que le ballon 40 est fixé sur le corps cylindrique 2 du cathéter à proximité immédiate du cône de l'extrémité 20, et plus particulièrement au niveau de la base la plus large du cône, c'est à dire celle la plus éloignée du bout du cathéter (figures 11, 12).

La bague 23 et le corps cylindrique 2 sont percés d'un premier trou débouchant 25 permettant à l'alésage 37 de l'entrée 31 de communiquer avec le canal de grand diamètre 21.

L'entrée 31 coopère avec un bouchon étanche, non représenté, permettant, par serrage de ce dernier sur la partie filetée de l'entrée 31, de réaliser l'étanchéité avec le mandrin ou l'instrument introduit dans le canal 21.

Ainsi, on remarque que deux entrées 30 et 31 débouchent dans le même canal 21 ménagé dans la partie interne du corps cylindrique 2.

De même, la bague 23 et le corps cylindrique 2 sont percés d'un autre trou débouchant 26 situé à l'opposé du premier 25 pour permettre à l'alésage 36 de l'entrée 32 de communiquer avec le canal de plus petit diamètre 22. Ce dernier, disposé parallèlement au canal 21, est prévu pour déboucher au niveau des moyens d'étanchéité 4 pour permettre de gonfler le ballon 40.

On note que le cathéter universel 1 décrit ci-dessus est du type trois entrées deux voies, c'est à dire que deux entrées débouchent dans le même canal.

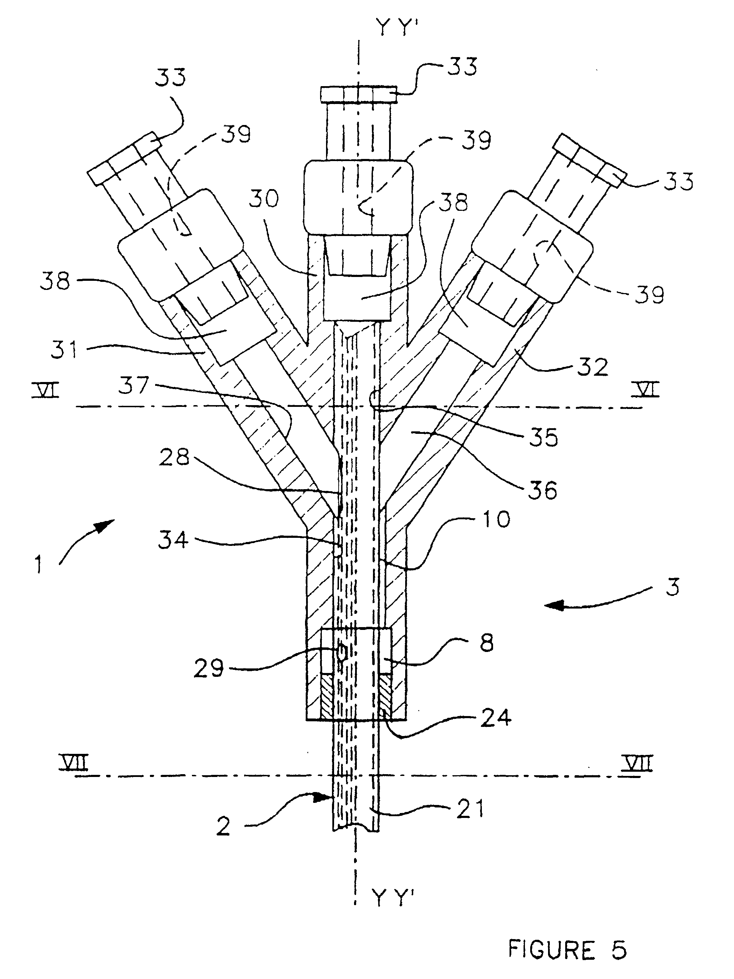

En figures 5 à 7 on a montré une seconde variante du cathéter universel 1 dont le corps cylindrique 2 comporte en plus des canaux 21 et 22, un autre canal 27 porté par des axes qui sont décalés latéralement par rapport à ceux principaux XX' et YY' du corps cylindrique et décalés par rapport à ceux portant les canaux 21 et 22.

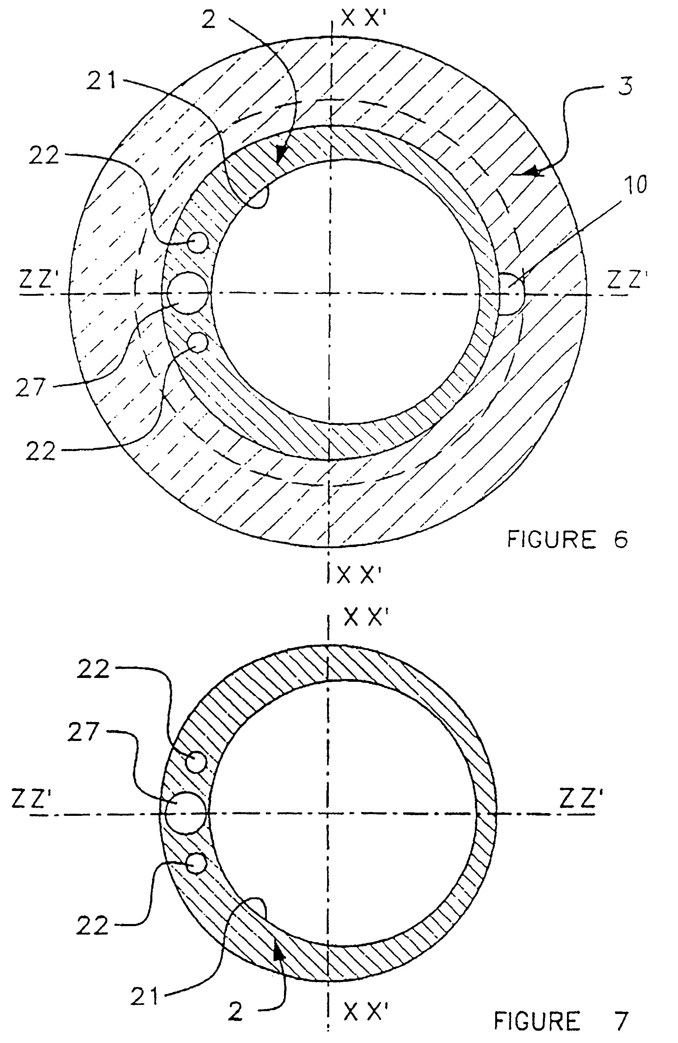

Dans cette variante il est prévu deux canaux 22 qui sont percés de part et d'autre du canal 27 entre ce dernier et le canal 21. On remarque que les canaux 22 sont portés par un même axe qui est parallèle à celui XX' du corps cylindrique 2, mais disposé dans un plan vertical différent comme montré en figure 7.

On constate que les canaux 21 et 27 sont portés par le même axe ZZ' du corps cylindrique 2 (figure 7).

Le corps cylindrique 2 est solidaire de la tête 3 décrite ci-dessus munie de ses trois entrées indépendantes 30, 31 et 32.

On remarque que les entrées 30, 31, 32 sont solidaires de l'élément de liaison 33, afin de délimiter la chambre 38.

Le corps cylindrique 2 est solidaire de la tête 3 au moyen de la bague 24, comme décrit précédemment, tandis que dans cette variante le diamètre extérieur du corps 2 est collé directement dans l'alésage 35.

On note que la bague 24 est de longueur inférieure à celle décrite précédemment pour délimiter dans l'épaulement de l'alésage 34 une chambre 8 qui est traversée par le corps cylindrique 2.

En effet, le retrait de la bague 23 permet de réaliser un canal longitudinal 10 (figures 5 et 6) ménagé dans l'alésage 34 de façon à faire communiquer la chambre 8 pour alimenter les canaux 22 pour le gonflage du ballon 40.

Le canal longitudinal 10 est prévu uniquement du côté de l'entrée 32, de manière que la paroi externe du corps cylindrique 2 soit en contact étanche, d'une part sur le pourtour interne de l'alésage 35, et d'autre part à l'opposé du canal longitudinal 10 sur le pourtour interne de l'alésage 34 pour que l'alésage 37 de l'entrée 31 ne puisse pas communiquer avec la chambre 8.

Au niveau de l'alésage 37 de l'entrée 31 le corps cylindrique 2 est percé d'un premier trou débouchant 28 qui permet la communication entre ledit alésage 37 et le canal 27.

Dans cette variante les canaux 21 et 27 débouchent à l'extérieur du côté opposée à la tête 3 et plus particulièrement au niveau de l'extrémité en forme de pointe effilée 20.

On note que le corps cylindrique 2 est percé au niveau de la chambre de trous débouchant 29 qui communiquent avec les canaux 22. Ainsi, l'entrée 32 est reliée par l'intermédiaire de son alésage 36, du canal longitudinal 10, de la chambre 8, des trous 29 et des canaux 22 avec le ballon 40 qui est disposé sur le corps cylindrique 2 à l'opposé de la tête 3, afin de le déformer élastiquement sous un effort de pression.

Le cathéter 1 décrit ci-dessus et montré en figures 5 à 7, comporte trois entrées et trois voies ou canaux par rapport au précèdent qui ne comportait que deux voies ou canaux.

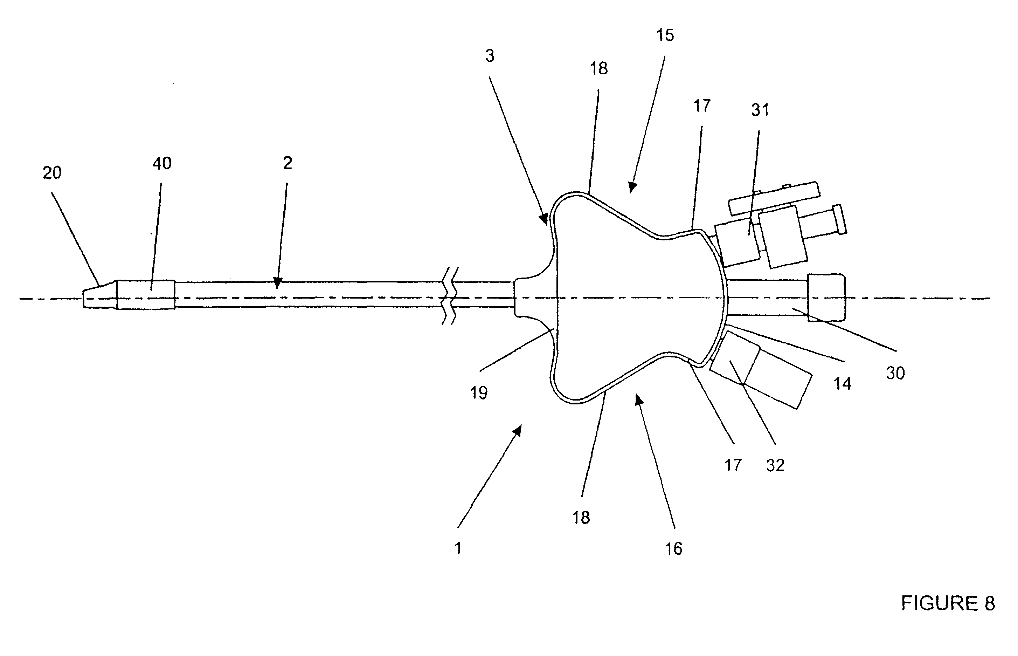

En figures 8 à 10 on a montré une troisième variante du cathéter universel 1 en ce qui concerne le profil de la tête de connexion 3 sur le corps cylindrique 2.

La tête de connexion 3 présente un profil très ergonomique permettant au chirurgien de bien maintenir le cathéter 1 entre les doigts d'une de ses mains lors des différentes interventions.

La tête 3 comporte une paroi 14 légèrement bombée recevant les trois entrées indépendantes 30, 31, 32. La paroi 14 est disposée perpendiculairement au corps cylindrique 2 et se prolonge de chaque côté dudit corps par une paroi 15, 16 à pans inclinés. Chaque paroi 15, 16 présente à proximité des entrées 31, 32 un pan incliné 17 de faible longueur qui est dirigé en direction du corps cylindrique 2. Chaque pan incliné 17 se prolonge par un autre pan incliné 18 d'inclinaison différente et dirigé de manière à s'éloigner du corps cylindrique 2. Chaque paroi inclinée 18 des parois 15, 16 se réunie au niveau du corps cylindrique 2 et à l'opposé de la paroi 14 par une paroi 19 incurvée en direction dudit corps.

Ainsi, le profil de la tête de connexion 3 permet une meilleur préhension de la part du chirurgien pour parfaitement introduire le corps cylindrique 2 dans les différents sites opératoires.

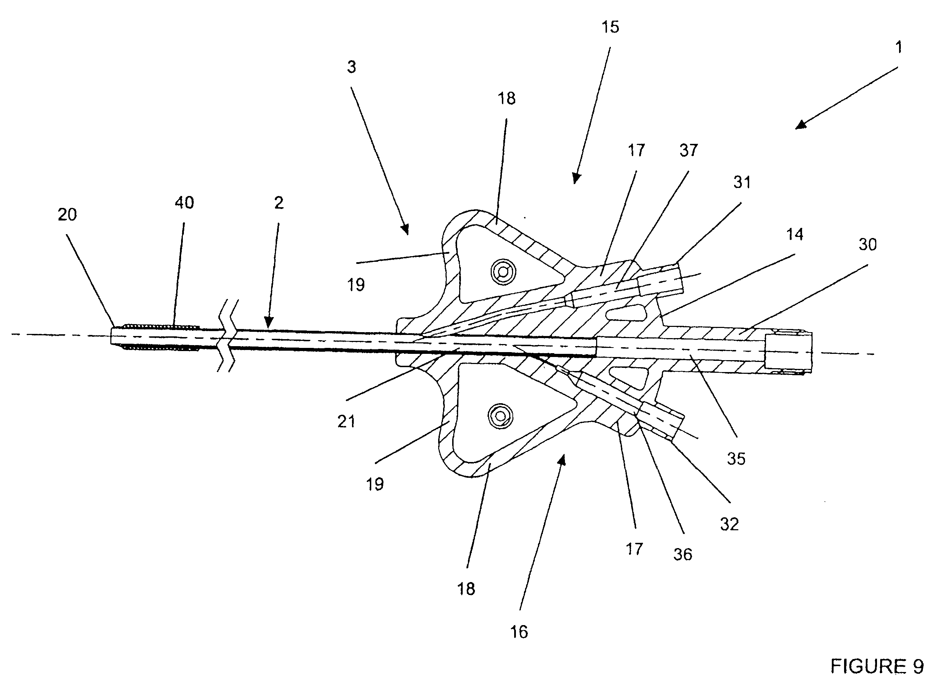

En figure 9 on a représenté la partie interne de la tête de connexion 3 dont les entrées 30, 31, 32 coopèrent avec les canaux non coaxiaux 21, 22 du corps cylindrique 2 comme cela a été décrit en figure 2 à 4.

Ainsi, la tête de connexion 3 permet à l'entrée 30 de communiquer avec le canal 21, dit canal principal, tandis que l'entrée 32 coopère avec le canal 22 pour alimenter le ballon 40.

On note que l'entrée 31 coopère également avec le canal 21 du corps cylindrique 2. On remarque que l'entrée 30 comprend un alésage interne 35 qui est désaxé pour venir coopérer en face du canal 21, étant donné que ce dernier est excentré par rapport au diamètre externe du corps cylindrique 2 (figure 4).

Dans cette réalisation de la tête de connexion 3, on constate que les chambres 38 on été supprimées de manière que chaque alésage 35, 36, 37 des entrées 30, 31, 32 débouche directement dans les canaux non coaxiaux du corps 2 et à l'extrémité de la tête 3.

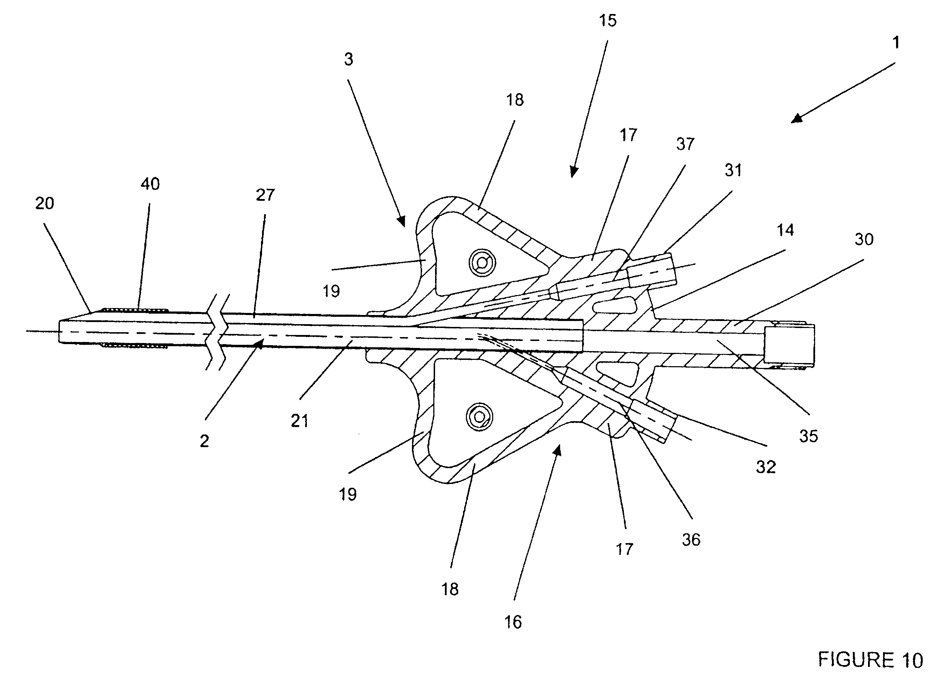

En figure 10 on a illustré la partie interne de la tête de connexion 3 dont les entrées 30, 31, 32 coopèrent avec les canaux non coaxiaux 21, 22, 27 du corps cylindrique 2 comme cela est décrit en figures 5 à 7.

Ainsi, la tête de connexion 3 permet à l'entrée 30 de communiquer avec le canal 21, tandis que les entrées 31 et 32 coopèrent respectivement avec le canal 27, dit canal opérateur, pour le passage d'instruments et les canaux 22 pour l'alimentation du ballon 40.

On remarque que l'entrée 31 présente un alésage interne 37 dont l'inclinaison permet la communication avec le canal 27 par l'intermédiaire d'un trou percé au travers du corps cylindrique 2.

Par contre, l'entrée 32 présente un alésage interne 36 qui est également incliné et tourné d'un quart de tour autour de l'axe du corps cylindrique 2 pour venir communiquer par l'intermédiaire de trous dans les canaux 22. Cette position permet de supprimer la chambre 8 et le canal longitudinal 10, montré en figure 5.

Dans les solutions décrites ci-dessus et représentées en figure 9 et 10, la tête de connexion 3 est réalisée en matière plastique injectée qui vient directement se fixer au moment du moulage autour du corps cylindrique 2, garantissant ainsi une parfaite étanchéité de la tête 3 sur le corps 2.

En figure 11, l'extrémité du corps cylindrique 2 est solidaire d'un ballon ou ballonnet 40 dont le profil extérieur dépend de la distance entre les points de fixation dudit ballonnet sur le corps 2.

Ainsi, on note que les zones de fixation du ballonnet 40 sont rapprochées permettant à ce dernier de présenter, lorsqu'il et gonflé, un profil très arrondi comme un pneu, autour du corps cylindrique 2.

En figure 12, les zones de fixation du ballonnet 40 sont plus éloignées que celles montrées précédemment, ce qui permet de définir un volume du ballonnet qui est différent lorsqu'il est gonflé.

Le ballonnet 40 prévu à l'extrémité du corps cylindrique 2 du cathéter 1 permet de réaliser une étanchéité en venant prendre appui contre la paroi du site opératoire, comme on le verra mieux plus loin. Également, le ballonnet 40 permet de réaliser un appui et une sorte de rotule facilitant les déplacements du cathéter 1 dans l'espace du site opératoire et empêche le rejet hors du site.

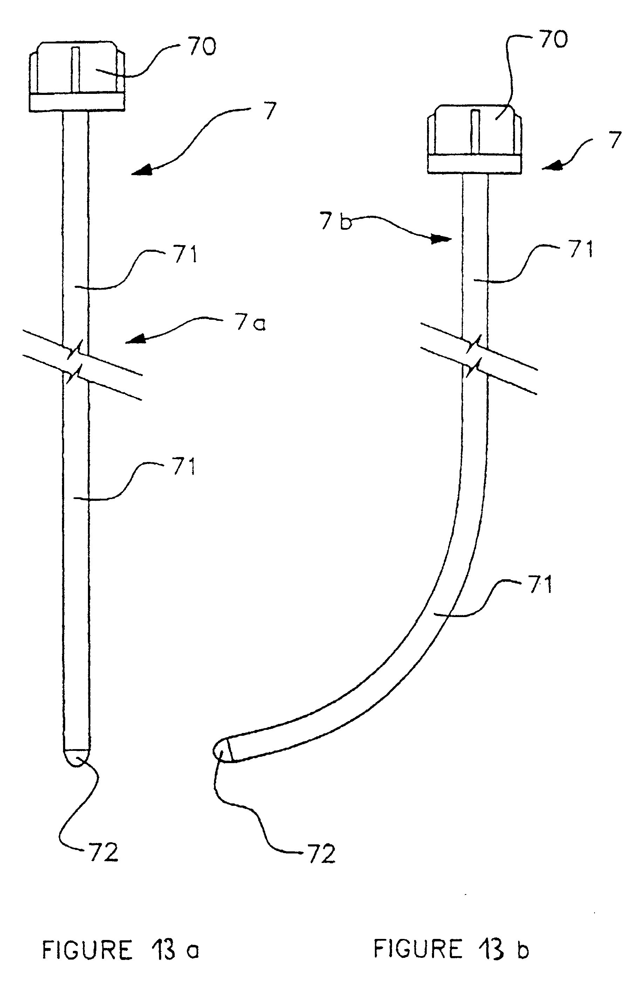

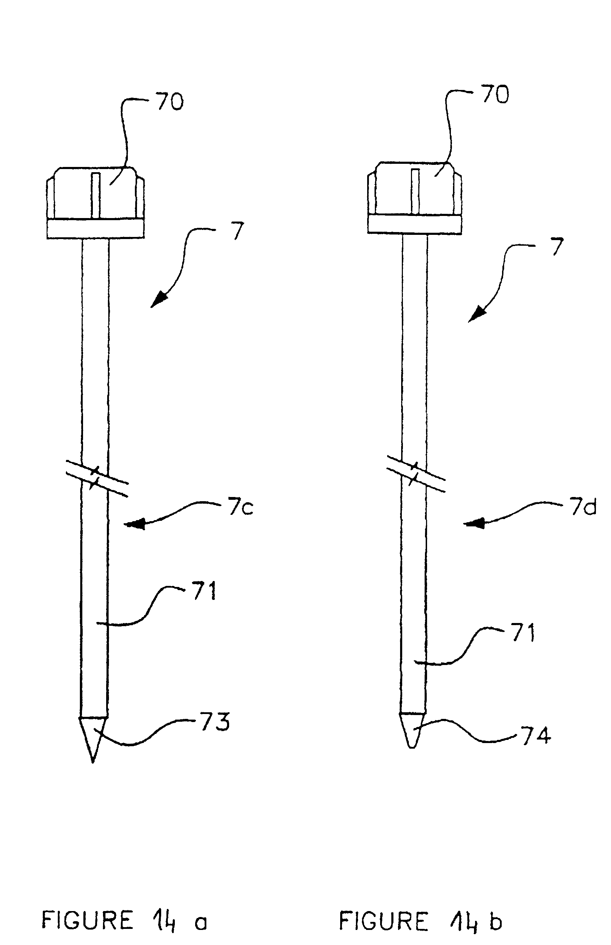

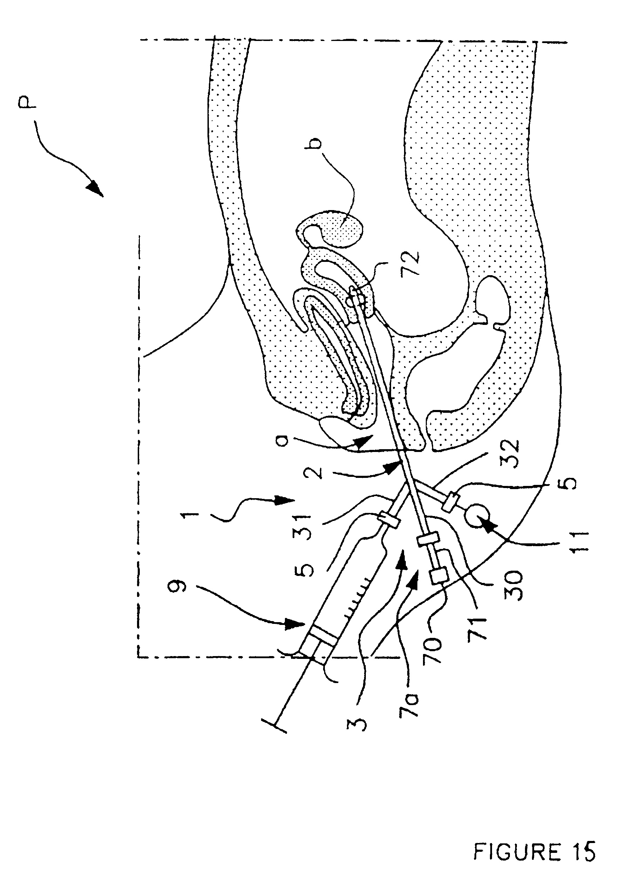

En figures 13a, 13b et 14a, 14b on a montré une série 7a, 7b, 7c, 7d d'instruments chirurgicaux, ou mandrins 7, permettant la réalisation des examens illustrés en figures 15 et 11.

Les mandrins 7 sont constitués dans chaque représentation d'une tête 70 en matière plastique solidaire d'une tige métallique 71 de faible diamètre.

On remarque que seule l'extrémité libre opposée à la tête 70 varie dans sa forme géométrique et sa matière pour permettre différent type examen.

En figure 13a le mandrin 7a présente au bout de sa tige 71 une extrémité libre 72 réalisée suivant un profil hémisphérique.

En figure 13b le mandrin 7b comporte une tige 70 présentant un profil courbe, mais dont l'extrémité libre 72 est réalisée suivant un profil hémisphérique.

En figure 14a le mandrin 7c comprend au bout de sa tige 70 une extrémité libre 73 en forme de pointe très effilée ou conique dont l'inclinaison est semblable à celle de l'extrémité 20 du corps cylindrique 2 pour venir se loger dans son prolongement (figure 11).

Enfin, en figure 14b le dernier mandrin 7d de la série comporte une extrémité libre 74 à profil conique, mais dont le bout est légèrement arrondi. De même que précédemment, le profil conique de l'extrémité 74 vient dans le prolongement de l'extrémité conique 20 du corps 2.

Le cathéter universel 1 décrit précédemment et ses mandrins 7a, 7b, 7c, 7d, sont conçus pour effectuer un ensemble de procédures réunies sous l'appellation FERTILOSCOPY et qui consiste en plusieurs étapes permettant :
- une appréciation de l'état utérin,
- une appréciation de la perméabilité tubaire,
- une appréciation de l'environnement péri tubo ovarien,
- une visualisation sans pareil de la partie distale des trompes et des ovaires,
- une appréciation de la qualité tubaire par la salpingoscopie associée.

La figure 15 illustre un premier exemple d'examen au moyen du cathéter universel 1 comportant une tête 3 à trois entrées et deux canaux 21 et 22.

Le cathéter 1 est introduit à l'intérieur de l'utérus a d'une patiente P afin de réaliser, par exemple, un test au bleu de méthylène afin de vérifier la perméabilité des trompes b.

La tête 3, et plus particulièrement l'entrée 31, est solidaire d'un robinet 5 associé à une seringue 9 remplie d'un liquide constitué de bleu de méthylène, tandis que l'entrée 30 est fermée de manière étanche par la tête 70 d'un mandrin 7.

L'entrée 32 est reliée par l'intermédiaire d'une vanne anti-retour à une source d'air ou de liquide sous pression 11 pour permettre de gonfler le ballon 40 à l'intérieur de l'utérus a afin d'obstruer ce dernier et de le rendre étanche lors de l'introduction du bleu de méthylène.

Les mandrins 7a ou 7b sont introduits par l'entrée 30 qui communique avec le même canal 21 que celui de l'entrée 31 utilisé pour l'introduction du bleu de méthylène afin que l'extrémité 72 soit logée dans le site opératoire de l'utérus a. L'introduction d'un mandrin 7a ou 7b permet la mobilisation de l'utérus a pour faciliter son examen lors de la coelioscopie effectuée conjointement.

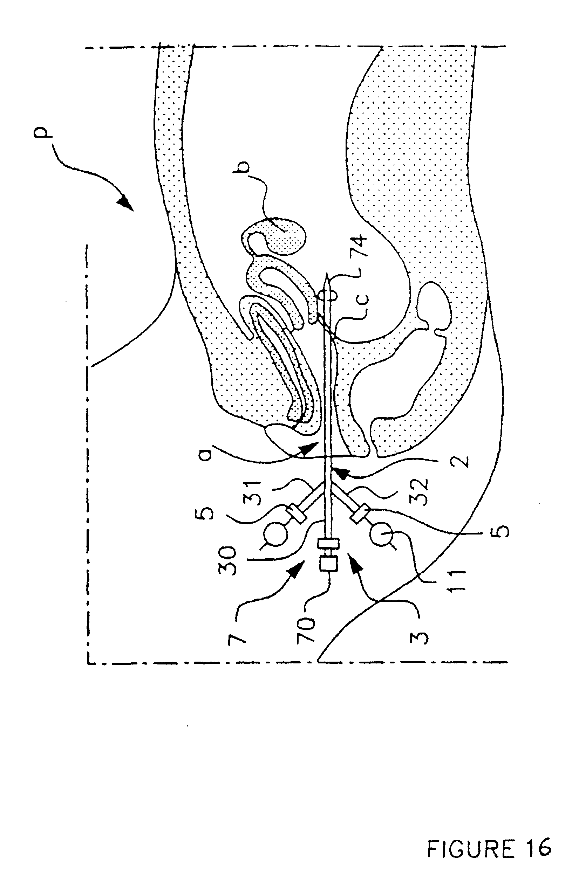

La figure 16 montre une autre intervention au moyen du cathéter universel 1 à trois entrées et deux canaux pour une FERTILOSCOPY.

Cette opération consiste à réaliser une incision du cul de sac vaginal c sous anesthésie locale. Cette FERTILOSCOPY est réalisée à l'aide du cathéter universel 1 associé avec un mandrin 7 tel que 7c qui est introduit dans le canal opératoire 27 du corps cylindrique 2.

La FERTILOSCOPY consiste à la création d'une ascite artificielle par du sérum qui est injecté au moyen du cathéter universel dans le site opératoire pour permettre l'observation des annexes dans les conditions les plus physiologiques possibles.

On peut également à l'aide du cathéter 1 à trois entrées et trois voies ou canaux, réaliser une FERTILOSCOPY en introduisant des mandrins chirurgicaux dans le canal 27 du corps cylindrique, et notamment une pince à préhension stabilisant le pavillon, un FERTILOSCOPE permettant de réaliser de manière indépendante une salpingoscopie.

Le même canal 27 permet de réaliser des biopsies, incision d'hydrosalpinx avant de prendre une décision opératoire.

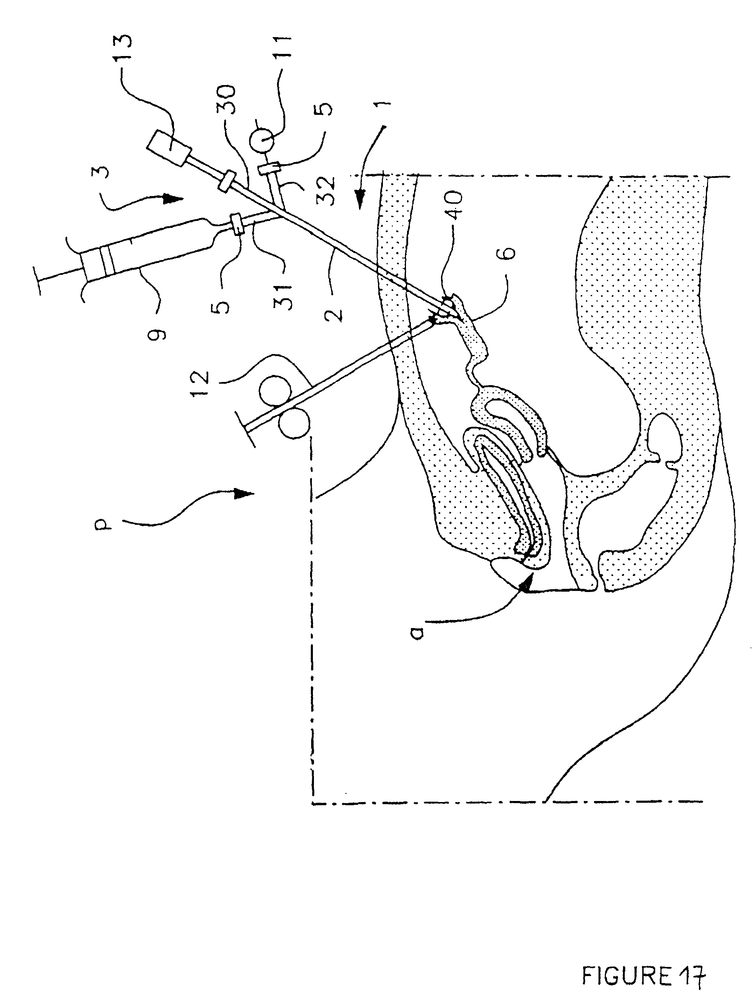

La figure 17 représente une autre application d'utilisation du cathéter universel 1 comme trocart au cours d'une coelioscopie pour une salpingoscopie.

Le cathéter universel 1 est introduit dans le pavillon d'une trompe b qui est soulevée par l'intermédiaire d'une pince 12. Le ballon 40 est gonflé au moyen d'une source d'air ou de liquide sous pression 11 qui est raccordée à l'entrée 32 par l'intermédiaire d'une vanne anti-retour 5. Un mini endoscope 13 est introduit par l'entrée 30 de manière que son optique soit logée à l'intérieur du pavillon à ausculté. Ce mini endoscope est placé après le retrait d'un mandrin 7c à extrémité 73 qui permet de percer la peau de la patiente P.

Une seringue 9 est disposée sur l'entrée 31 qui comporte un autre robinet 5 afin de remplir le pavillon de sérum facilitant l'examen optique.

Après avoir retiré le mini endoscope 13, la salpingotomie peut être réalisée si nécessaire en s'aidant du ballon 40.

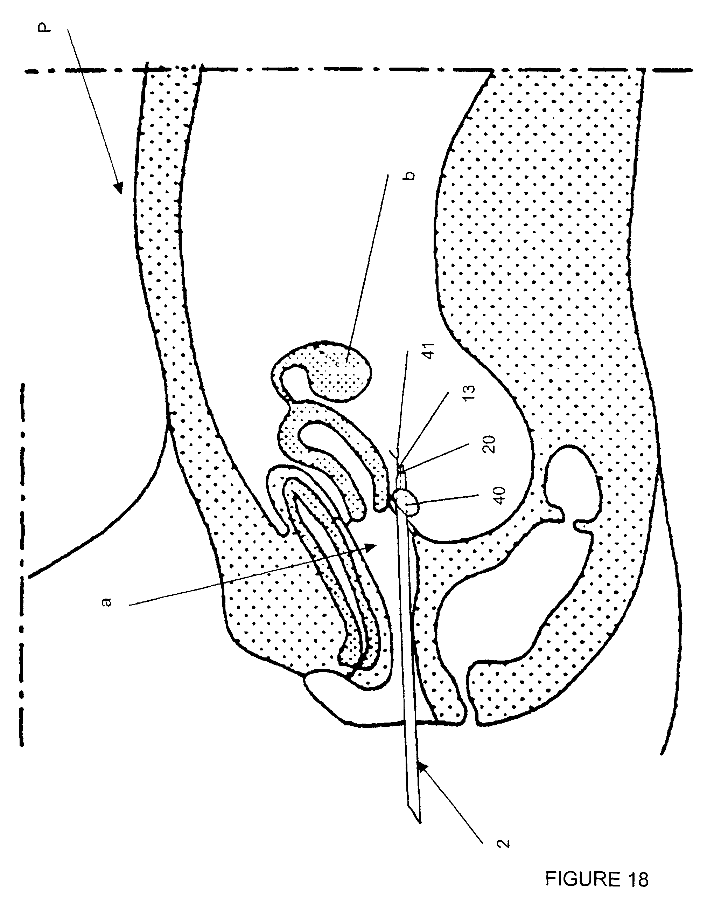

La figure 18 illustre une dernière application d'utilisation du cathéter universel 1 pour réaliser une salpingoscopie. Le cathéter utilisé est un trois entrées trois voies, permettant, lorsqu'il est introduit dans le site opératoire, d'amener par le canal opérateur 27 une pince 41, tandis qu'un mini endoscope 13 est disposé dans le canal excentré 21. Les dimensions de ce dernier permettent d'injecter un liquide autour du mini endoscope 13 pendant la visite de la trompe retenue par la pince.

On remarque que les diamètres des canaux 21 et 27 sont adaptés pour recevoir les tiges 71 des mandrins 7a, 7b, 7c, 7d,ou d'instruments chirurgicaux tout en permettant l'injection d'un liquide dans le même canal.

On constate que le cathéter universel 1, lorsqu'il est utilisé en trocart, remplace la gaine d'infiltration de liquide qui est obligatoire lors de l'utilisation des mini endoscopes optiques 13. En outre, le diamètre du canal 21 permet de recevoir tout mini endoscope 13 dont le diamètre extérieur n'excède pas 4mm lorsque la gaine ou chemise d'infiltration est retirée.

De plus, le corps cylindrique 2 est réalisé dans une matière plastique transparente permettant le contrôle de la descente de l'optique 13 à l'intérieur du cathéter 1.

On remarque que le cathéter universel suivant la présente invention, se substitue aux gaines ou chemises nécessaires à l'usage des mini endoscopes.

Enfin on remarque que le cathéter universel 1 est plus couramment utilisé dans le domaine gynécologique pour réaliser l'ensemble des examens effectués à ce jour.

## Revendications

1. Cathéter universel pour permettre différentes interventions comprenant un corps cylindrique (2) de faible diamètre comportant à l'une de ses extrémités des moyens de connexion (3) qui communiquent avec des canaux (21, 22, 27) non coaxiaux ménagés dans la partie interne dudit corps cylindrique (2) de sorte que l'un des canaux (22) alimente, à l'extrémité opposée à celle portant les moyens de connexion, un ballonnet (40) susceptible de se déformer élastiquement, tandis que deux autres canaux (21, 27) permettent l'introduction, soit de liquide, soit d'une gamme, de mandrins chirurgicaux (7), soit les deux en même temps, au niveau du site opératoire, **caractérisé en ce que** les moyens de connexion sont constitués d'une tête monobloc (3) réalisée en matière plastique rigide qui peut être infectée pour venir se fixer de manière étanche autour du corps cylindrique (2), ladite tête (3) comportant une paroi bombée (14) pourvue de trois entrées indépendantes (30, 31, 32) qui communiquent par l'intermédiaire d'alésages (35, 36, 37) avec les canaux (21, 22, 27) non coaxiaux, deux parois (15, 16) à pans inclinés prolongeant la paroi (14) de chaque coté du corps cylindrique (2), lesdites parois (15, 16) présentant chacune des pans (17, 18) opposés d'inclinaison différente, et d'une paroi (19) à profil incurvé raccordant les pans (18) de chaque paroi (15, 16) au niveau du corps cylindrique (2), de manière que la tête (3) présente un profil extérieur général ergonomique facilitant la préhension du cathéter.

2. Cathéter universel suivant la revendication 1, **caractérisé en ce que** chaque paroi (15, 16) présente à proximité des entrées indépendantes (31, 32) un pan incliné (17) de faible longueur qui est dirigé en direction du corps cylindrique (2), tandis que chaque pan (17) se prolonge par un autre pan incliné (18) qui est dirigé de manière à s'éloigner du corps cylindrique (2) pour venir se raccorder avec la paroi incurvée (19).

3. Cathéter universel suivant la revendication 1, **caractérisé en ce que** le corps cylindrique (2) présente à l'opposé de la tête monobloc (3) une extrémité (20) à profil conique.

4. Cathéter universel suivant la revendication 3, **caractérisé en ce que** le profil conique de l'extrémité (20) du corps cylindrique (2) est axé sur le canal principal (21).

5. Cathéter universel suivant la revendication 3, **caractérisé en ce que** le canal principal (21) est excentré par rapport au diamètre extérieur du corps cylindrique (2).

6. Cathéter universel suivant la revendication 3, **caractérisé en ce que** le ballonnet (40) est fixé sur le corps cylindrique (2) a proximité immédiate du cône de l'extrémité (20) et plus particulièrement au niveau de la base la plus large du cône, c'est à dire celle la plus éloignée du bout du cathéter (1).

7. Cathéter universel suivant la revendication 1, **caractérisé en ce que** les moyens de connexion sont constitués d'une tête (3) réalisée en matière plastique rigide munie de trois entrées indépendantes (30, 31, 32) qui débouchent chacune dans un alésage interne (34) par l'intermédiaire d'alésages (35, 36, 37), lesdits alésages (34, 35) étant prévus cour recevoir par l'intermédiaire de moyens étanches (23, 24) le profil longitudinal externe du corps cylindrique (2) de manière que lesdits alésages (35, 36, 37) de chaque entrée indépendante communiquent par des trous (25, 26, 28, 29) qui traversent le corps cylindrique (2) et/ou les moyens étanches (23, 24) avec les canaux non coaxiaux (21, 22, 27) afin que les canaux (21, 27) permettent l'introduction, soit de produits liquides, soit d'une gamme de mandrins chirurgicaux (7) et/ou autres instruments (13, 41), soit les deux en même temps au niveau des différents sites opératoires.

8. Cathéter universel suivant la revendication 7, **caractérisé en ce que** les moyens étanches sont constitués d'une première bague (24) qui est disposée autour du corps cylindrique (2) à l'opposé de l'entrée (30) dans un épaulement de l'alésage (34), et d'une seconde bague (23) qui est placée autour dudit corps cylindrique (2) dans le prolongement de la première de manière à coopérer avec les alésages (34, 35) de la tête (3).

9. Cathéter universel suivant la revendication 7, **caractérisé en ce que** les moyens étanches sont constitués d'une bague (24) qui est disposée autour du corps cylindrique (2) à l'opposé de l'entrée (30) dans un épaulement de l'alésage (34), tandis que le diamètre extérieur du corps (2) est directement assujetti dans l'alésage (35) de la tête (3).

10. Cathéter universel suivant la revendication 81, **caractérisé en ce que** les bagues (23, 24) sont collées sur le profil externe du corps cylindrique (2) et sur le pourtour interne des alésages (34, 35) afin de rendre la tête (3) solidaire dudit corps.

11. Cathéter universel suivant la revendication 9, **caractérisé en ce que** la bague (24) présente une longueur déterminée pour délimiter dans l'épaulement de l'alésage (34) une chambre (8) qui communique avec l'alésage (36) de l'entrée (32) de la tête (3) par l'intermédiaire d'un espace (10).

12. Cathéter universel suivant la revendication 11, **caractérisé en ce que** l'espace (10) est prévu uniquement du côté de l'entrée (32) de la tête (3), tandis que la paroi externe du corps cylindrique (2) est en contact étanche, d'une part avec le pourtour interne de l'alésage (35), et d'autre part à l'opposé de l'espace (10) avec le pourtour interne de l'alésage (34).

13. Cathéter universel suivant l'une quelconque des revendications 1 et 7, **caractérisé en ce que** le corps cylindrique (2) est percé de deux canaux (21, 22) non coaxiaux de diamètres différents qui sont décalés l'un par rapport à l'autre et latéralement par rapport aux axes principaux (XX', YY') dudit corps de manière que le premier canal (21) communique avec deux entrées (30, 31 ) de la tête (3), tandis que le second canal coopère avec la troisième entrée (32) pour l'alimentation des moyens d'étanchéité (4).

14. Cathéter universel suivant l'une quelconque des revendications 1 et 7, **caractérisé en ce que** le corps cylindrique (2) est percé d'au moins trois canaux (21, 22, 27) non coaxiaux de diamètres différents qui sont décalés les uns par rapport aux autres et latéralement par rapport aux axes principaux (XX', YY') dudit corps de manière que le premier canal (21) de plus grand diamètre communique avec la première entrée (30) de la tête (3), le second canal (22) avec la troisième entrée (32) de la tête (3) pour l'alimentation des moyens d'étanchéité (4), et le troisième canal (27) avec la seconde entrée (31) pour déboucher à l'extérieur du corps (2) au même niveau que le premier canal (21).

15. Cathéter universel suivant l'une quelconque des revendications 1 et 7, **caractérisé en ce que** le corps cylindrique (2) est percé d'un trou (25, 28) pour permettre la communication entre le canal (21, 27) et l'alésage (37) de l'entrée (31).

16. Cathéter universel suivant l'une quelconque des revendications 1 et 7, **caractérisé en ce que** le corps cylindrique (2) est percé d'un trou (26) permettant la communication entre le canal (22) et l'alésage (36) de l'entrée (32).

17. Cathéter universel suivant l'une quelconque des revendications 1 et 7, **caractérisé en ce que** le corps cylindrique (2) est percé de deux canaux (22) qui communiquent par l'intermédiaire de trous (29) dans la chambre (8) reliée à un espace (10) pour déboucher dans l'alésage (36) de la seconde entrée (32) de la tête (3) afin d'alimenter les moyens étanches (4).

18. Cathéter universel suivant l'une quelconque des revendications 1 et 7, **caractérisé en ce que** les mandrins chirurgicaux (7a, 7b, 7c, 7d) comportent une tête (70) solidaire d'une tige (71) dont l'extrémité libre (72, 73, 74) varie dans sa forme géométrique et sa matière.

19. Cathéter universel suivant la revendication 18, **caractérisé en ce que** le mandrin (7a) présente une extrémité libre (72) réalisée suivant un profil hémisphérique.

20. Cathéter universel suivant la revendication 18, **caractérisé en ce que** le mandrin (7b) présente une tige (71 ) à profil courbe dont l'extrémité libre (72) est réalisée suivant un profil hémisphérique.

21. Cathéter universel suivant l'une quelconque des revendications 1 et 7, **caractérisé en ce que** le corps cylindrique (2) est rigide.

22. Cathéter universel suivant l'une quelconque des revendications 1 et 7, **caractérisé en ce que** le corps cylindrique (2) est souple.

23. Cathéter universel suivant l'une quelconque des revendications 1 et 7, **caractérisé en ce que** le corps cylindrique (2) est transparent.

## Claims

1. Multipurpose catheter intended to permit various interventions, comprising a cylindrical body (2) of small diameter which has, at one of its ends, connection means (3) which communicate with non-coaxial channels (21, 22, 27) formed in the inner part of the said cylindrical body (2) in such a way that one of the channels (22) supplies, to the end remote from that bearing the connection means, a balloon (40) which is able to deform elastically, while two other channels (21, 27) permit introduction either of liquid or of a range of surgical mandrins (7), or both simultaneously, at the operating site, **characterized in that** the connection means consist of a monobloc head (3) made of rigid plastic material which can be injection-molded to attach itself in a sealed manner around the cylindrical body (2), the said head (3) including a bulged wall (14) provided with three independent inlets (30, 31, 32) which communicate with the non-coaxial channels (21, 22, 27) via bores (35, 36, 37), two walls (15, 16) with inclined surfaces continuing the wall (14) on each side of the cylindrical body (2), the said walls (15, 16) each having opposite surfaces (17, 18) of different inclination, and a wall (19) of inwardly curved profile connecting the surfaces (18) of each wall (15, 16) in the area of the cylindrical body (2), in such a way that the head (3) has a general ergonomic external profile facilitating the gripping of the catheter.

2. Multipurpose catheter according to Claim 1, **characterized in that** each wall (15, 16) has, in proximity to the independent inlets (31, 32), an inclined surface (17) of short length which is oriented in the direction of the cylindrical body (2), while each surface (17) is continued by another inclined surface (18) which is oriented away from the cylindrical body (2) to join up with the inwardly curved wall (19).

3. Multipurpose catheter according to Claim 1, **characterized in that** the cylindrical body (2) has, remote from the monobloc head (3), an end (2) with a conical profile.

4. Multipurpose catheter according to Claim 3, **characterized in that** the conical profile of the end (20) of the cylindrical body (2) has its axis on the main channel (21).

5. Multipurpose catheter according to Claim 3, **characterized in that** the main channel (21) is eccentric in relation to the external diameter of the cylindrical body (2).

6. Multipurpose catheter according to Claim 3, **characterized in that** the balloon (40) is fixed on the cylindrical body (2) in immediate proximity to the cone of the end (20) and more particularly at the widest base of the cone, that is to say the one furthest from the end of the catheter (1).

7. Multipurpose catheter according to Claim 1, **characterized in that** the connection means consist of a head (3) made of rigid plastic material and equipped with three independent inlets (30, 31, 32) which each open into an internal bore (34) via bores (35, 36, 37), the said bores (34, 35) being provided to receive through sealed means (23, 24) the external longitudinal profile of the cylindrical body (2) such that the said bores (35, 36, 37) of each independent inlet communicate via holes (25, 26, 28, 29) passing through the cylindrical body (2) and/or the sealed means (23, 24) with the non-coaxial channels (21, 22, 27) so that the channels (21, 27) permit introduction either of liquid products or of a range of surgical mandrins (7) and/or other instruments (13, 41), or both simultaneously at the different operating sites.

8. Multipurpose catheter according to Claim 7, **characterized in that** the sealed means consist of a first ring (24) which is arranged around the cylindrical body (2) opposite the inlet (30) in a shoulder of the bore (34), and a second ring (23) which is placed around the said cylindrical body (2) in the continuation of the first, in such a way as to cooperate with the bores (34, 35) of the head (3).

9. Multipurpose catheter according to Claim 7, **characterized in that** the sealed means consist of a ring (24) which is arranged around the cylindrical body (2) opposite the inlet (30) in a shoulder of the bore (34), while the external diameter of the body (2) is directly fixed in the bore (35) of the head (3).

10. Multipurpose catheter according to Claim 81 [sic], **characterized in that** the rings (23, 24) are bonded on the external profile of the cylindrical body (2) and on the internal periphery of the bores (34, 35) in order to make the head (3) integral with the said body.

11. Multipurpose catheter according to Claim 9, **characterized in that** the ring (24) has a length which is defined in order to delimit, in the shoulder of the bore (34), a chamber (8) which communicates with the bore (36) of the inlet (32) of the head (3) via a space (10).

12. Multipurpose catheter according to Claim 11, **characterized in that** the space (10) is provided solely on the inlet side (32) of the head (3), while the outer wall of the cylindrical body (2) is in tight contact on the one hand with the internal periphery of the bore (35) and, on the other hand, opposite the space (10), with the internal periphery of the bore (34).

13. Multipurpose catheter according to either of Claims 1 and 7, **characterized in that** the cylindrical body (2) has two non-coaxial channels (21, 22) of different diameters which are offset in relation to each other and laterally in relation to the main axes (XX', YY') of the said body, in such a way that the first channel (21) communicates with two inlets (30, 31) of the head (3), while the second channel cooperates with the third inlet (32) for supplying the sealing means (4).

14. Multipurpose catheter according to either of Claims 1 and 7, **characterized in that** the cylindrical body (2) has at least three non-coaxial channels (21, 22, 27) of different diameters which are offset in relation to each other and laterally in relation to the main axes (XX', YY') of the said body, in such a way that the first channel (21) of greater diameter communicates with the first inlet (30) of the head (3), the second channel (22) with the third inlet (32) of the head (3) for supplying the sealing means (4), and the third channel (27) with the second inlet (31) for emerging from the body (2) at the same level as the first channel (21).

15. Multipurpose catheter according to either of Claims 1 and 7, **characterized in that** the cylindrical body (2) has a hole (25, 28) to permit communication between the channel (21, 27) and the bore (37) of the inlet (31).

16. Multipurpose catheter according to either of Claims 1 and 7, **characterized in that** the cylindrical body (2) has a hole (26) permitting communication between the channel (22) and the bore (36) of the inlet (32).

17. Multipurpose catheter according to either of Claims 1 and 7, **characterized in that** the cylindrical body (2) has two channels (22) which communicate via holes (29) in the chamber (8) connected to a space (10) in order to open into the bore (36) of the second inlet (32) of the head (3) in order to supply the sealed means (4).

18. Multipurpose catheter according to either of Claims 1 and 7, **characterized in that** the surgical mandrins (7a, 7b, 7c, 7d) include a head (70) integral with a rod (71) whose free end (72, 73, 74) varies in its geometric shape and its material.

19. Multipurpose catheter according to Claim 18, **characterized in that** the mandrin (7a) has a free end (72) designed with a hemispherical profile.

20. Multipurpose catheter according to Claim 18, **characterized in that** the mandrin (7b) has a rod (71) of curved profile whose free end (72) is designed with a hemispherical profile.

21. Multipurpose catheter according to either of Claims 1 and 7, **characterized in that** the cylindrical body (2) is rigid.

22. Multipurpose catheter according to either of Claims 1 and 7, **characterized in that** the cylindrical body (2) is flexible.

23. Multipurpose catheter according to either of Claims 1 and 7, **characterized in that** the cylindrical body (2) is transparent.

## Patentansprüche

1. Universalkatheter zum Gestatten von unterschiedlichen Eingriffen mit einem zylindrischen Körper (2) mit kleinem Durchmesser, der an einem seiner Enden Verbindungsmittel (3) umfasst, die mit nicht koaxialen Kanälen (21, 22, 27) in Verbindung stehen, die so im inneren Teil des zylindrischen Körpers (2) angeordnet sind, dass einer (22) der Kanäle an dem Ende, das dem die Verbindungsmittel tragenden Ende gegenüberliegt, einen Ballon (40) speist, der sich elastisch verformen kann, während die beiden anderen Kanäle (21, 27) das Einführen von Flüssigkeit oder von verschiedenen chirurgischen Dornen (7) oder von beiden gleichzeitig zur Operationsstelle gestatten, **dadurch gekennzeichnet, dass** die Verbindungsmittel aus einem einstückigen Kopf (3) bestehen, der aus starrem Kunststoff hergestellt ist, der so eingespritzt werden kann, dass er sich dicht um den zylindrischen Körper (2) legt, wobei der Kopf (3) eine bauchige Wand (14) umfasst, die mit drei unabhängigen Einlässen (30, 31, 32), die über Bohrungen (35, 36, 37) mit den nicht koaxialen Kanälen (21, 22, 27) in Verbindung stehen, zwei Wänden (15, 16) mit geneigten Flächen, die die Wand (14) auf jeder Seite des zylindrischen Körpers (2) fortsetzen, wobei die Wände (15, 16) jeweils gegenüberliegende Flächen (17, 18) mit unterschiedlicher Neigung aufweisen, und einer Wand (19) mit nach innen gekrümmtem Profil versehen ist, die die Flächen (18) jeder Wand (15, 16) in Höhe des zylindrischen Körpers (2) so miteinander verbindet, dass der Kopf (3) ein ergonomisches allgemeines äußeres Profil aufweist, das das Greifen des Katheters erleichtert.

2. Universalkatheter nach Anspruch 1, **dadurch gekennzeichnet, dass** jede Wand (15, 16) in der Nähe der unabhängigen Einlässe (31, 32) eine geneigte Fläche (17) von geringer Länge aufweist, die in Richtung des zylindrischen Körpers (2) geneigt ist, während jede Fläche (17) durch eine andere geneigte Fläche (18) fortgesetzt ist, die so ausgerichtet ist, dass sie sich vom zylindrischen Körper (2) entfernt und an die nach innen gekrümmte Wand (19) anschließt.

3. Universalkatheter nach Anspruch 1, **dadurch gekennzeichnet, dass** der zylindrische Körper (2) gegenüber dem einstückigen Kopf (3) ein Ende (20) mit einem konischen Profil aufweist.

4. Universalkatheter nach Anspruch 3, **dadurch gekennzeichnet, dass** die Achse des konischen Profils des Endes (20) des zylindrischen Körpers (2) auf dem Hauptkanal (21) liegt.

5. Universalkatheter nach Anspruch 3, **dadurch gekennzeichnet, dass** der Hauptkanal (21) zum Außendurchmesser des zylindrischen Körpers (2) exzentrisch verläuft.

6. Universalkatheter nach Anspruch 3, **dadurch gekennzeichnet, dass** der Ballon (40) in unmittelbarer Nähe des Kegels des Endes (20) am zylindrischen Körper (2) befestigt ist, und zwar im Einzelnen an der breitesten Basis des Kegels, d.h. derjenigen, die am weitesten vom Ende des Katheters (1) entfernt ist.

7. Universalkatheter nach Anspruch 1, **dadurch gekennzeichnet, dass** die Verbindungsmittel aus einem Kopf (3) aus einem starren Kunststoffmaterial mit drei unabhängigen Einlässen (30, 31, 32) bestehen, die jeweils über Bohrungen (35, 36, 37) in eine innere Bohrung (34) münden, wobei die Bohrungen (34, 35) dazu vorgesehen sind, über dichte Mittel (23, 24) das äußere Längsprofil des zylindrischen Körpers (2) so aufzunehmen, dass die Bohrungen (35, 36, 37) jedes unabhängigen Einlasses über Löcher (25, 26, 28, 29), die durch den zylindrischen Körper (2) und/oder die dichten Mittel (23, 24) gehen, mit den nicht koaxialen Kanälen (21, 22, 27) in Verbindung stehen, so dass die Kanäle (21, 27) das Einführen von Flüssigkeiten oder von verschiedenen chirurgischen Dornen (7) und/oder von anderen Instrumenten (13, 41) oder von beiden gleichzeitig zu verschiedenen Operationsstellen gestatten.

8. Universalkatheter nach Anspruch 7, **dadurch gekennzeichnet, dass** die dichten Mittel aus einem ersten Ring (24), der um den zylindrischen Körper (2) gegenüber dem Einlass (30) in einen Absatz der Bohrung (34) angeordnet ist, und einem zweiten Ring (23) bestehen, der um den zylindrischen Körper (2) in der Fortsetzung des ersten Rings so angeordnet ist, dass er mit den Bohrungen (34, 35) des Kopfs (3) zusammenwirkt.

9. Universalkatheter nach Anspruch 7, **dadurch gekennzeichnet, dass** die dichten Mittel aus einem Ring (24) bestehen, der um den zylindrischen Körper (2) gegenüber dem Einlass (30) in einen Absatz der Bohrung (34) angeordnet ist, während der Außendurchmesser des Körpers (2) direkt in der Bohrung (35) des Kopfs (3) befestigt ist.

10. Universalkatheter nach Anspruch 8, **dadurch gekennzeichnet, dass** die Ringe (23, 24) auf das Außenprofil des zylindrischen Körpers (2) und auf den Innenumfang der Bohrungen (34, 35) aufgeklebt sind, damit der Kopf (3) fest mit dem Körper verbunden ist.

11. Universalkatheter nach Anspruch 9, **dadurch gekennzeichnet, dass** der Ring (24) eine bestimmte Länge hat, um in dem Absatz der Bohrung (34) eine Kammer (8) zu definieren, die über einen Raum (10) mit der Bohrung (36) des Einlasses (32) des Kopfs (3) in Verbindung steht.

12. Universalkatheter nach Anspruch 11, **dadurch gekennzeichnet, dass** der Raum (10) nur auf der Seite des Einlasses (32) des Kopfs (3) vorgesehen ist, während die Außenwand des zylindrischen Körpers (2) einerseits mit dem Innenumfang der Bohrung (35) und andererseits gegenüber dem Raum (10) mit dem Innenumfang der Bohrung (24) in dichtem Kontakt steht.

13. Universalkatheter nach Anspruch 1 oder 7, **dadurch gekennzeichnet, dass** der zylindrische Körper (2) zwei nicht koaxiale Kanäle (21, 22) mit unterschiedlichen Durchmessern aufweist, die zueinander und seitlich zu den Hauptachsen (XX', YY') des Körpers so versetzt sind, dass der erste Kanal (21) mit zwei Einlässen (30, 31) des Kopfs (3) in Verbindung steht, während der zweite Kanal mit dem dritten Einlass (32) zusammenwirkt, um die Dichtungsmittel (4) zu speisen.

14. Universalkatheter nach Anspruch 1 oder 7, **dadurch gekennzeichnet, dass** der zylindrische Körper (2) mindestens drei nicht koaxiale Kanäle (21, 22, 27) mit unterschiedlichen Durchmessern aufweist, die zueinander und seitlich zu den Hauptachsen (XX', YY') des Körpers so versetzt sind, dass der erste Kanal (21) mit dem größeren Durchmesser mit dem ersten Einlass (30) des Kopfs (3) und der zweite Kanal (22) mit dem dritten Einlass (32) des Kopfs (3) in Verbindung steht, um die Dichtungsmittel (4) zu speisen, und der dritte Kanal (27) mit dem zweiten Einlass (31) in Verbindung steht und auf gleicher Höhe wie der erste Kanal (21) außerhalb des Körpers (2) mündet.

15. Universalkatheter nach Anspruch 1 oder 7, **dadurch gekennzeichnet, dass** der zylindrische Körper (2) ein Loch (25, 28) aufweist, damit eine Verbindung zwischen zwischen dem Kanal (21, 27) und der Bohrung (37) des Einlasses (31) besteht.

16. Universalkatheter nach Anspruch 1 oder 7, **dadurch gekennzeichnet, dass** der zylindrische Körper (2) ein Loch (26) aufweist, damit eine Verbindung zwischen zwischen dem Kanal (22) und der Bohrung (36) des Einlasses (32) besteht.

17. Universalkatheter nach Anspruch 1 oder 7, **dadurch gekennzeichnet, dass** der zylindrische Körper (2) zwei Kanäle (22) aufweist, die über Löcher (29) in der mit einem Raum (10) verbundenen Kammer (8) miteinander in Verbindung stehen und in der Bohrung (36) des zweiten Einlasses (32) des Kopfs (3) münden, um die dichten Mittel (4) zu speisen.

18. Universalkatheter nach Anspruch 1 oder 7, **dadurch gekennzeichnet, dass** die chirurgischen Dorne (7a, 7b, 7c, 7d) einen Kopf (70) umfassen, der fest mit einer Stange (71) verbunden ist, deren freies Ende (72, 73, 74) von unterschiedlicher geometrischer Form und aus unterschiedlichem Material ist.

19. Universalkatheter nach Anspruch 18, **dadurch gekennzeichnet, dass** der Dorn (7a) ein freies Ende (72) aufweist, das mit einem halbkugelförmigen Profil ausgeführt ist.

20. Universalkatheter nach Anspruch 18, **dadurch gekennzeichnet, dass** der Dorn (7b) eine Stange (71) mit gebogenem Profil aufweist, deren freies Ende (72) mit einem halbkugelförmigen Profil ausgeführt ist.

21. Universalkatheter nach Anspruch 1 oder 7, **dadurch gekennzeichnet, dass** der zylindrische Körper (2) starr ist.

22. Universalkatheter nach Anspruch 1 oder 7, **dadurch gekennzeichnet, dass** der zylindrische Körper (2) flexibel ist.

23. Universalkatheter nach Anspruch 1 oder 7, **dadurch gekennzeichnet, dass** der zylindrische Körper (2) transparent ist.
